# EUROPEAN PATENT APPLICATION

(11) **EP 1 270 042 A1**
(43) Date of publication of application: **02.01.2003**
(21) Application number: 01924030.8
(22) Date of filing: 27.03.2001
(51) Int. Cl.: A61N 1/365, A61N 1/36

(54) **IMPLANTED AND PROGRAMMABLE ELECTROSTIMULATOR FOR STIMULATING ORGANS AND TISSUES OF AN ORGANISM**

(30) Priority: 29.03.2000 RU 2000107563
(71) Applicant: Patent Max Q (P.M.Q) Ltd., Road Town, Tortola (VG)
(72) Inventor: KARASHUROV, Sergei Egorovich, Petrozavodsk, 185001 (RU)
(74) Representative: Waxweiler, Jean
(86) International application number: RU0100126
(87) International publication number: WO01074446

(57) **Abstract**

The invention relates to medical technology. Implanted programmable electrostimulator comprises an autonomous power supply unit, an A/D converter of signals emitted by biosensors (3a, 3b), a programmable unit for forming output pulses arranged in body (1), wire-electrode (2) placed outside body (1) and used for pulsing the organs and tissues. According to the invention the electrostimulator comprises a radio-frequency unit for communicating with external programming and power supplying device (5) placed inside body (1) and biosensors (3a) controlling the functional condition of respiratory system (respiratory output) and biosensors (3b) for controlling the functional condition of cardio-vascular system (cardio-vascular output). The electrostimulator can be powered by autonomous unit and by external programming and power supplying device (5).

## Description

### FIELD OF THE INVENTION

The invention related to medical technology and in particular to implanted electrostimulators designated for permanent or pulsating stimulation of organs and tissues of an organism by electric pulses.

### BACKGROUND OF THE INVENTION

USSR Patent N 1627189 A1 with application priority 15.02.91 discloses implanted Electrostimulator for stimulating of nervous tissue of an organism and it comprises a transmitting device and a receiving device. The transmitting device comprises a generator of stimulating pulses, the first differentiating element, the first duration generator, the first amplifier, a switching device, an inductor, a frequency divider, the second differentiating element, the second duration generator and the second amplifier. The receiver comprises communication coil enclosed in a capsule with conductors and electrodes. The drawbacks of the known device: such device cannot be automatically adjusted to suit the changing needs of the organism, does not include sensitive elements providing for interconnection between the stimulation parameters and the body condition, it has only radio-frequency power supplying unit which impairs its reliability, enables stimulating only nervous tissue.

Application PCT/US92/05980 with application priority 22.07.91 discloses implanted electrostimulator having autonomous power supplying unit, A/D converter of signals emitted by biosensor, programmable unit for forming output pulses arranged in a body, as well as wire-electrode placed outside the body and used pulsing organs and tissues and a biosensor.

The drawback of the known device: low reliability and short service life because of autonomous power supplying unit being the only power supplying device, insufficient sensitivity and electrostimulator efficiency because of only one respiratory output biosensor.

### SUMMARY OF THE INVENTION

The object of the invention is to improve reliability and service life of the electrostimulator, to improve its efficiency, to enable automatic setting of optimal parameters of organs and tissues stimulation.

To attain the above objects of the invention implanted programmable electrostimulation of organs and tissues comprises autonomous power supply unit, A/D converter of signals emitted by biosensor, programmable unit for forming of output pulses arranged in the body and wire-conductor used for pulsing the organs and tissues and biosensors placed outside the body, according to the invention it comprises a radio frequency unit for communicating with external programming and power supplying unit arranged in the body and connected with programmable unit used for forming output pulses, biosensors controlling the respiratory output and cardio-vascular output connected to A/D converter which is in turn connected to a programmable unit used for output pulses forming and supplying output pulses to wire-electrode connected to it, moreover the radio frequency communication unit, the programmable unit used for forming output pulses and the A/D converted are connected to autonomous power supplying unit too.

Electrostimulator according to the invention has an outer shell made from porous bio-inert material over the body and wire-electrode with biosensors placed inside the bio-inert material pores. Biosensors controlling the respiratory output and biosensors controlling the cardio-vascular output are spread over the entire surface of the outer shell. Great quantity of biosensors and their arrangement over the entire outer surface of the electrostimulator provides for more precise control of the respiratory and cardio-vascular systems as well as for the possibility for automatic adjustment of the electrostimulator parameters in order to suit the organism needs, and in this way improves the electrostimulator efficiency.

Electrostimulator in accordance with the invention can be powered by an autonomous power-supplying unit as well as by an external programming and power-supplying device via a radio frequency communication unit.

The availability of two power supplying sources - autonomous power supply and an external programming and power-supplying unit via radio frequency communication unit improves the reliability and service life of the electrostimulator.

The electrostimulator design in accordance with the invention enables receiving the needed output pulse parameters by means of radio frequency communication unit from the external programming device and power supply, programming the algorithm of forming of output pulses in accordance with the signal parameters emitted by the biosensors controlling the respiratory outputs and cardio-vascular outputs, and enables their transmission to the wire-electrode.

In the electrostimulator design in accordance with the invention the radio frequency communication unit has two communication channels with the external programming and power supplying unit, one of which enables data transmission needed for programming of the electrostimulator output pulse parameters and operation modes, energy transmission for powering the electrostimulator and charging its autonomous power-supplying unit, and the other one enables to apply stimulation pulses from the external programming and power supplying unit directly to wire-electrode in case of the electrostimulator failure.

In the electrostimulator design in accordance with the invention the radio frequency communication unit enables controlling the stimulation pulse parameters in the wire -electrode by means of the external programming and power-supplying unit.

In the electrostimulator design in accordance with the invention the A/D converter has two separate channels, one used by the biosensors controlling the respiratory output and the other one - by the biosensors controlling cardio-vascular output.

### BRIEF DESCRIPTION OF THE DRAWINGS

The inventions is described with the reference to the accompanying drawings:
Fig. 1 is a general view of the electrostimulator according to the invention;
Fig. 2 is a block-diagram of the electrostimulator according to the invention.

### DESCRIPTION OF THE PREFERRED EMBODIMENTS

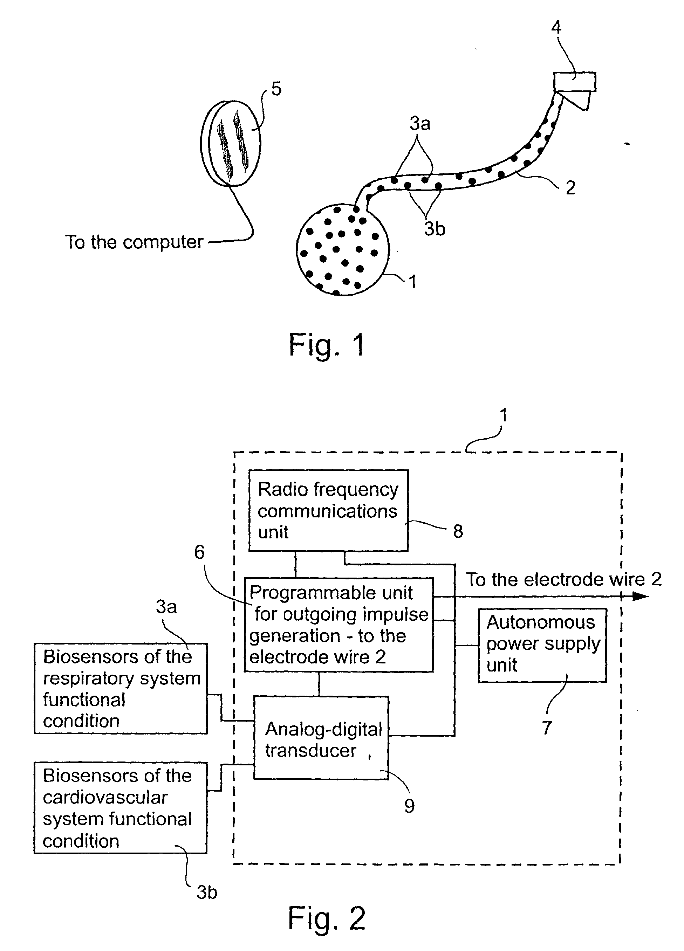

Fig. 1 is a general view of implanted programmable electrostimulator. The main electrostimulator units are arranged in body 1. Wire -electrode 2 is connected to electrostimulator body 1 by multi-contact connector. Electrostimulator body 1 and wire-electrode 2 are coated with external shell made from porous bio-inert material, such as porous silicone and biosensors (3a) controlling respiratory output and biosensors (3b) controlling cardio-vascular output are positioned in its pores. Contacts 4 at the end of wire-electrode 2 are connected to the stimulated tissue or organ. Electrostimulator body 1 is implanted subcutaneous, for example on the front surface of the thorax, or more commonly on the subclavian area or in the front abdominal wall, more commonly to subcostal area. Wire-electrode 2 is passed through a subcutaneous tunnel specially formed with a surgery tool in the part of the body where the nerve is located and is connected to it with contacts 4. Wire-electrode 2 must not necessarily be connected to nervous tissue.

Biosensors 3a, 3b are mounted in the external shell pores by electrochemical method. The most suitable biosensors 3a, 3b are carbon sensors in which the carbon particles are coated with low-dispersed current-conducting silicone. The amount of biosensors 3a, 3b may be in range from 40 to 20000. Biosensors 3a controlling the respiratory output and biosensors 3b controlling cardio-vascular output are evenly spread over the surface of body 1 and wire-conductor 2 external shell. Design of biosensors 3a controlling the respiratory output is different from that of biosensors 3b controlling cardio-vascular output. In particular, they have different thickness of contact membranes, different structure of chemical reagent positioned underneath the membrane and inside it.

Where electrostimulator body 1 is implanted, external programming and power supplying device 5 is positioned on the skin.

Fig. 2 is electrostimulator block diagram. The heart of the electrostimulator is programmable unit 6 used for forming the output pulses built around a microprocessor. This unit 6 is connected to autonomous power supply unit 7, to radio frequency communication unit 8 with external programming and power supplying device 5 and t A/D converter 9. A/D converter 9 is connected with biosensors 3a controlling the respiratory output and biosensors 3b controlling cardio-vascular output, and it has two separate channels, one for biosensors 3a controlling the respiratory output and the other one for biosensors 3b controlling cardio-vascular output. Radio frequency communication unit 8, programmable unit 6 used to for forming of output pulses and A/D converter 9 are connected to autonomous power supply unit 7. Programmable unit 6 used for forming output pulses is connected to wire-electrode 2 via multi-contact connector in order to apply stimulating pulses to contacts 4 of wire-electrode 2.

In accordance with the developed algorithm programmable unit 6 forms the output pulse parameters depending on signals emitted by biosensors 3a, 3b via A/D converter 9. The biosensor 3a and 3b signals depend on the oxygen contents, catecholamines contents in blood, respiratory murmur, respiration rate and pulse frequency. In A/D converter 9 the signals are separated to signals characterizing the cardio-vascular output and signals characterizing the respiratory output by rate since the signal rate is much higher than the respiration rate.

Programmable unit 6sued for forming of output pulses is programmed so that when such parameters of signals emitted by biosensors 3a, 3b as frequency, voltage, current are increased the output pulse parameters such as current, voltage pulse duration, the intervals between pulses are reduced, or the other way round, as the mentioned parameters of signals emitted by biosensors are decreased the above parameters of output pulses are increased. Programmable unit 6 programs the output pulse parameters by means of external programming and power supplying device 5 connected to it through radio frequency communication unit 8.

Programmable output pulses forming unit 6 may receive output pulse parameters via radio frequency communication unit 8 from external programming and power supplying device 5, which programs the algorithm of forming the output pulses in accordance with the parameters of signals emitted by biosensors 3a controlling respiratory output and biosensors 3b controlling cardio-vascular output and then transfer them to wire-electrode 2.

Programmable output pulses forming unit 6, radio frequency communication unit 8 and A/D converter 9 may be powered either from autonomous power supplying unit 7 or from external programming and power supplying device 5 via radio frequency communication unit 8.

Radio-frequency unit 8 for communicating with external programming and power supply device 5 has two communication channels. One channel is used for data transfer to programmable unit 6 used for forming output pulses for programming the electrostimulator output pulses and operation modes and for energy transfer for powering the electrostimulator and charging its autonomous power supplying unit 7. The second communication channel is used for direct conveying of the stimulation pulses from external programming and power supply device 5 to wire-electrode 2 in case of the electrostimulator failure. The stimulation pulse parameters in wire-electrode 2 can be controlled also by the external programming and power supply device 5 via radio frequency communication unit 8.

Electrostimulator forms pulses with frequency from 0.01q Hz to 1000 Hz, duration of 0.01-5.0ms, amplitude of 0.1-15.0V, as well as pulse bursts with adjustment range of bursts duration and intervals between them from 0.01 to 24 hours.

Electrostimulator can be switched on and off by the external programming and power supply device 5 via radio frequency communication unit 8.

The electrostimulator operates as follows. Changes in cardio-vascular and respiratory systems resulting from a disease are registered by the electrostimulator biosensors. Further, in accordance with the developed electrostimulation algorithm the electrostimulator forms current pulses in wire-electrode 2, which are applied to the affected object - tissue or organ, and return the respiratory and cardio-vascular system parameters to their normal values by changing their functional activity.

## Claims

1. Implanted programmable electrostimulator for stimulating organs and tissues of an organism comprises an autonomous power supply unit, an A/D converter of signals emitted by biosensors, a programmable block for forming output pulses arranged in a body and a wire-electrode placed outside-the body and used for pulsing the organs and tissues and biosensors, **characterized in that** is contains radio-frequency unit for communicating with external programming and power supply device placed in the body and connected to programmable unit used for forming of output pulses, biosensors controlling respiratory output and biosensors controlling cardio-vascular output, connected by A/D converter connected to programmable unit used for output pulses forming supplying output pulses to wire-electrode connected to it, moreover the radio frequency communication unit, the programmable output pulses forming unit and the A/D converter are connected also to the autonomous power supplying unit.

2. Electrostimulator according to claim 1, **characterized in that** it has an external shell made from porous material coating the body and the wire-electrode moreover biosensors are mounted in the porous material pores.

3. Electrostimulator according to claim 1 or 2, **characterized in that** the biosensors controlling respiratory output and biosensors controlling cardio-vascular output are spread over the entire surface of the external shell.

4. Electrostimulator according to claim 1, **characterized in that** it can be powered either from the autonomous power supplying unit or from an external programming and power supply device via radio frequency communication unit.

5. Electrostimulator according to claim 1, **characterized in that** output pulse parameters can be programmed by means of external programming and power supplying device connected to it via radio frequency communication unit.

6. Electrostimulator according to claim 1 or 5, **characterized in that** it enables receiving output pulses parameters via external programming and power supplying device, programming the algorithm of forming the output pulses in accordance with the signal parameters emitted by the biosensors controlling respiratory output and biosensors controlling a cardio-vascular output and their transmission to the wire-electrode.

7. Electrostimulator according to claim 1, **characterized in that** radio-frequency unit for communicating with external programming and power supply device has two communication channels, one of which enables transfer of data needed for programming the parameters of the electrostimulator output pulses and operation modes, energy transfer for electrostimulator feeding and charging its autonomous power supplying unit, and the other one enables applying stimulation pulses from the external programming and power supplying unit directly to wire-electrode in case of the electrostimulator failure.

8. Electrostimulator according to claim 7, **characterized in that** radio- frequency communication unit enables controlling the stimulation pulse parameters in wire-electrode by means of the external programming and power supplying unit.

9. Electrostimulator according to claim 7, **characterized in that** it can be switched on and off by means of the external programming and power supplying unit via radio frequency unit.

10. Electrostimulator according to claim 1, **characterized in that** the A/D converter has two separate channels, one of which is designated for the biosensors controlling respiratory output and the other one - for the biosensors for controlling a cardio-vascular output.
